(19) Europäisches Patentamt
European Patent Office
Office européen des brevets

(11) Numéro de publication: **0 092 628**
**B1**

(12) # FASCICULE DE BREVET EUROPEEN

(45) Date de publication du fascicule du brevet: **29.05.85**

(51) Int. Cl.[4]: **C 07 C 45/00,** C 07 C 49/813, C 07 C 49/807

(21) Numéro de dépôt: **82401219.9**

(22) Date de dépôt: **30.06.82**

(54) **Procédé de préparation de phénylcétones à partir d'halogéno ou trihalogénométhylbenzènes et de composés aliphatiques ou aromatiques trihalogénométhyles.**

(30) Priorité: **22.04.82 FR 8206904**

(43) Date de publication de la demande:
**02.11.83 Bulletin 83/44**

(45) Mention de la délivrance du brevet:
**29.05.85 Bulletin 85/22**

(84) Etats contractants désignés:
**AT BE CH DE FR GB IT LI LU NL SE**

(56) Documents cités:
**EP-A-0 043 861**
**DE-A-2 451 037**
**DE-C-876 690**
**US-A-2 974 172**
**US-A-4 207 266**

**Le dossier contient des informations techniques présentées postérieurement au dépôt de la demande et ne figurant pas dans le présent fascicule.**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526 Chemin du Bois, F-69140 Rillieux (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de phénylcétones à partir d'halogéno ou trihalogénométhylbenzènes et de composés aliphatiques ou aromatiques trihalogénométhylés.

On connait dans l'art antérieur des procédés de préparation de ce type.

C'est ainsi que G. OLAH décrit dans »Friedel—Crafts and Related Reaction III — Part I — 1964. Interscience Publishers« la réaction d'un dérivé aliphatique ou aromatique trihalogénométhylé avec un halogéno ou trihalogénométhylbenzène en présence de catalyseurs comme $AlCl_3$, $AlBr_3$, $FeCl_3$, $SbCl_5$ en milieu solvant organique, le substrat pouvant être également le solvant, le produit obtenu étant ensuite hydrolysé pour obtenir la cétone recherchée.

Ces procédés présentent des inconvénients qui tiennent essentiellement à la nature du catalyseur. En effet, il est nécessaire d'utiliser une quantité importante de ce catalyseur car ce dernier se complexe avec le groupement trihalogénométhyl du composé aromatique ou aliphatique et avec le produit résultant de la première étape. Cette quantité importante d'$AlCl_3$ entraine la mise en oeuvre d'une grande quantité d'eau pour son élimination. De plus sa récupération est impossible industriellement.

La demanderesse a maintenant découvert un procédé palliant les inconvénients des procédés de l'art antérieur.

La présente invention concerne un procédé de préparation de phénylcétones caractérisé en ce que, dans une première étape, on fait réagir un halogéno ou trihalogénométhylbenzène ayant pour formule générale:

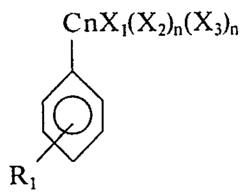

(I)

où:

- n est un nombre entier égal à 0 ou 1,
- $X_1$, $X_2$ et $X_3$ identiques ou différents représentent le chlore, le brome, l'iode ou le fluor et
- $R_1$ représente au moins un élément choisi parmi le groupe comprenant l'hydrogène, les radicaux alkyle et alkoxy ayant de 1 à 6 atomes de carbone, les radicaux phényle et phénoxy substitués par au moins un groupement plus désactivant que le groupement $CnX_1(X_2)_n(X_3)_n$, OH, Cl, Br, I et F

avec un composé aliphatique ou aromatique trihalogénométhylé en présence de trifluorure de bore en quantité telle que la pression absolue de trifluorure de bore dans l'enceinte réactionnelle soit supérieure à 1 bar et en présence d'acide fluorhydrique comme solvant et, en ce que, dans une deuxième étape, on hydrolyse le produit résultant.

On entend au sens de la présente invention par halogénobenzène ou trihalogénométhylbenzène d'une part ces produits proprement dits et d'autre part leurs homologues correspondant à la présence d'un ou plusieurs radicaux substituant le noyau benzénique.

Les radicaux $R_1$ phényle et phénoxy doivent être substitués par des groupements plus désactivants que le groupement $CnX_1(X_2)_n(X_3)_n$ pour que la réaction d'acylation intervienne sur le noyau benzénique porteur du groupement $CnX_1(X_2)_n(X_3)_n$. Dans le cas contraire, la réaction d'acylation se ferait sur le radical phényle ou phénoxy. On peut citer comme exemples de groupements plus désactivants que le groupement $CnX_1(X_2)_n(X_3)_n$, les groupements $NO_2$, COOH, CN et cétones.

Quand $n = 1$, on préfère mettre en oeuvre des composés de formule I pour lesquels $X_1$, $X_2$ et $X_3$ sont identiques. Parmi ces derniers, on préfère ceux pour lesquels $X_1$, $X_2$ et $X_3$ représentent le fluor.

On peut citer comme exemples de composés (I): le chlorobenzène, le fluorobenzène, le bromobenzène, l'iodobenzène, le trifluorométhylbenzène, le difluorobromométhylbenzène, le trichlorométhylbenzène, le dichlorofluorométhylbenzène, le tribromométhylbenzène, le dibromofluorométhylbenzène, le triodométhylbenzène, l'o-, m- et p-fluorotoluène, l'o-, m- et p-dichlorobenzène, l'o-, m- et p-fluorophénol, l'o-, m- et p-fluorochlorobenzène, l'o-, m- et p-fluoroanisole, l'o-, m- et p-difluorobenzène, l'o-, m- et p-chlorotoluène, l'o-, m- et p-chloroanisole, le trifluorométhyl-4 chloro-4 biphényle, le trifluorométhyl-4 dichloro-2,4' diphényle oxyde.

On entend au sens de la présente invention par composé aliphatique ou aromatique trihalogénométhylé, un composé de formule:

$$R_2{-}CX_4X_5X_6 \qquad (II)$$

où $R_2$ est un radical aliphatique ou aromatique et $X_4$, $X_5$ et $X_6$, identiques ou différents représentent Br, Cl ou F.

L'invention est particulièrement bien adaptée à la mise en oeuvre d'un composé de formule II dans laquelle $R_2$ représente un radical alkyle, phényle, alkylphényle, phénylalkyle ou phényle comportant au moins un substituant, comme par exemple, un halogène, $NO_2$, CN, $NH_2$, COOH.

On peut citer comme exemples de composés de formule II: trichloro-1,1,1 éthane, trichlorométhylbenzène, trifluorométhylbenzène, parafluorotrichlorométhylbenzène, parachlorotrifluorométhylbenzène, parachlorotrichlorométhylbenzène, orthochlorotrichlorométhylbenzène, métanitrotrichlorométhylbenzène, dichloro-3,4 trichlorométhylbenzène.

La première étape du procédé selon l'invention est mis en oeuvre de préférence en utilisant une quantité d'acide fluorhydrique telle que le rapport molaire de l'acide fluorhydrique à l'halogéno ou trihalogénomethylbenzène est compris entre 5 et 50. Encore plus préférentiellement, ce rapport est compris entre 10 et 30.

L'acide fluorhydrique mis en oeuvre est de préférence anhydre. La mise en oeuvre d'acide fluorhydrique aqueux entrainerait une consommation inutile de trifluorure de bore sous forme HF, $BF_3$, $H_2O$ ($H_3O^+BF_4^-$).

L'halogéno ou trihalogénométhylbenzène et les composés de formule II sont utilisés en quantité sensiblement équimolaires. Un excès du second peut cependant être souhaitable afin de minimiser la formation des composés de polycondensation.

On préfère plus particulièrement utiliser une quantité de trifluorure de bore telle que la pression initiale absolue de $BF_3$ dans l'enceinte réactionnelle soit comprise entre 6 et 20 bars. Une pression supérieure à 20 bars n'est pas exclue de l'invention mais n'apporte pas d'avantages particuliers. Plus la pression sera élevée, plus la vitesse de réaction augmentera. L'homme de l'art adaptera donc la pression à l'économie du procédé.

Lorsque l'on met en oeuvre un composé I ou II trichlorométhylé on observe une augmentation de pression due à l'échange Cl—F.

La première étape du procédé de l'invention est de préférence mise en oeuvre à une température comprise entre −20° C et 150° C.

Les temps de réaction sont généralement compris entre quelques minutes et plusieurs heures.

La deuxième étape est une hydrolyse qui peut être conduite en milieu acide ou basique comme cela est classique.

Une manière pratique de mettre en oeuvre le procédé selon l'invention est d'effectuer l'hydrolyse sur le mélange brut ou partiellement débarrassé du solvant HF issu de la première étape. On sera donc en présence d'HF donc en milieu acide.

L'élimination totale d'HF antérieurement à la deuxième étape permet d'opérer soit en milieu basique soit en milieu acide.

L'hydrolyse se fait de préférence, à une température comprise entre 0° C et 80° C-

Le procédé selon l'invention peut être schématisé de la façon suivante.

Quand n = 1

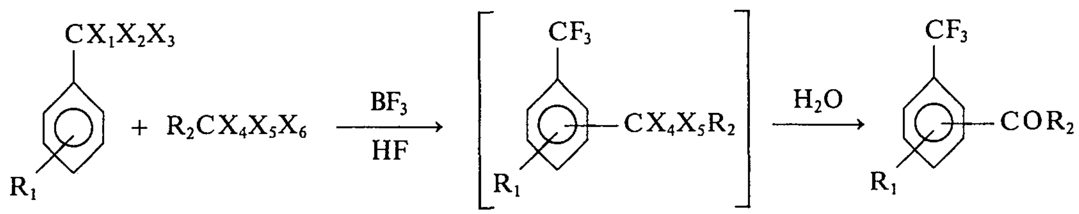


Quand n = 0

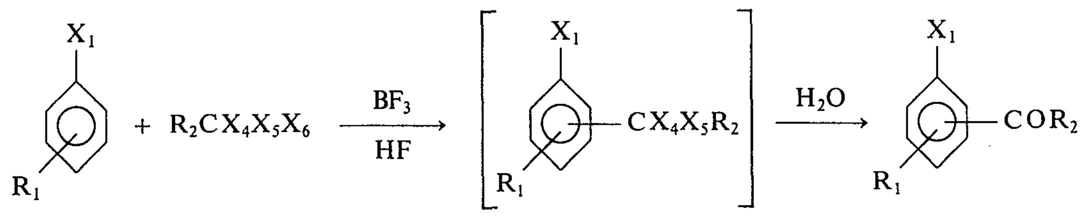


Les groupements $CCl_3$, $CBr_3$, $CI_3$, $CF_2Br$, $CCl_2F$, $CBr_2F$ etc. se transforment en $CF_3$ lors de la réaction en milieu HF, les substituants Cl, Br et I pour leur part ne s'échangeant pas.

La position du groupement $COR_2$ par rapport aux groupements $CF_3$, $X_1$ et $R_1$ obéit aux règles de substitution bien connues du chimiste organicien.

Les phénylcétones obtenues selon le procédé de l'invention sont utiles comme intermédiaires de synthèse de composés ayant une activité pharmaceutique ou phytosanitaire.

On peut citer comme exemples de composés pouvant être préparés par le procédé selon l'invention: fluoro-4 acétophénone, chloro-4 acétophénone, fluoro-2 méthyl-5 benzophénone, fluoro-3 méthyl-6 benzophénone, dichloro-2,4′ benzophénone, dichloro-2,4′-benzophénone, chloro-4 bromo-4 benzophénone, fluoro-4 bromo-4 benzophénone, difluoro-4,4′ benzophénone, trifluorométhyl-4 fluoro-4′ benzophénone, difluoro-4,4′ méthyl-3 benzophénone, difluoro-4,4′ méthoxy-3 benzophénone, fluoro-2 chloro-2′ méthyl-5 benzophénone, fluoro-3 chloro-2′ méthyl-6 benzophénone, fluoro-2 chloro-4′ méthyl-5 benzophénone, fluoro-3 chloro-4 méthyl-6 benzophénone, fluoro-4 chloro-4′ méthyl-3 benzophénone, trifluorométhyl-2 fluoro-2′ méthyl-5 benzophénone, trifluorométhyl-2 fluoro-3′ méthyl-6′ benzophénone.

L'invention va être maintenant plus complètement décrite à l'aide des exemples qui vont suivre. Ceux-ci sauraient être considérés comme limitant de façon quelconque l'invention.

## Exemple 1

Dans un réacteur inox de 250 ml muni d'un système d'agitation magnétique, on introduit aux environs de 0°C, 100 ml d'HF anhydre, 42,7 g (0,2 mole) de p-fluorotrichlorométhylbenzène et 22 g (0,2 mole) d'orthofluorotoluène.

Le réacteur est fermé puis on introduit du trifluorure de bore gazeux jusqu'à la pression constante de 6 bars. On laisse alors réagir sous agitation pendant 3 heures à la température ambiante. Après réaction, on décomprime le réacteur jusqu'à pression atmosphérique puis on coule le mélange réactionnel sur 200 g de glace pilée. Après réchauffement à température ambiante, le mélange hétérogène ainsi obtenu est agité pendant une à deux heures, puis extrait par trois fois 200 $cm^3$ de chlorure de méthylène. Les phases organiques sont lavées par trois fois 200 $cm^3$ d'eau, une fois 200 $cm^3$ d'une solution aqueuse à 3% de potasse et deux fois 200 $cm^3$ d'eau. La phase organique ainsi traitée est séchée sur sulfate de magnésium et le solvant est éliminé par distillation sous pression réduite.

On recueille ainsi 35,3 g (rendement: 61,5%) de difluoro-4,4′ methyl-3 benzophénone brute.

## Exemple 2

On procède comme à l'exemple 1 avec les composés et conditions suivants:

| | |
|---|---|
| — Acide fluorhydrique anhydre: | 100 g |
| — Trichlorométhylbenzène: | 19,6 g 0,1 mole |
| — Fluorobenzène: | 9,6 g 0,1 mole |
| — Trifluorure de bore: | 6 bars à 20°C |
| — Température: | 50°C |
| — Durée: | 4 heures |

On recueille 18,5 g (rendement: 92,5%) de fluoro-4 benzophénone brute.

## Exemple 3

On procède comme à l'exemple 1 avec les composés et conditions suivants:

| | |
|---|---|
| — Acide fluorhydrique anhydre: | 100 g |
| — p-chlorotrifluorométhylbenzène: | 18 g 0,1 mole |
| — Chlorbenzène: | 11,2 g 0,1 mole |
| — Trifluorure de bore: | 6 bars à 20°C |
| — Température: | 80°C |
| — Durée: | 15 heures |

On recueille 21 g (rendement: 89,4%) d'un mélange de dichloro-2,4′ et dichloro-4,4′ benzophénone brute.

## Exemple 4

On procède comme à l'exemple 1 avec les composés et conditions suivants:

| | |
|---|---|
| — Acide fluorhydrique anhydre: | 100 g |
| — p-chlorotrichlorométhylbenzène: | 23 g 0,1 mole |
| — Chlorobenzène: | 11,2 g 0,1 mole |
| — Trifluorure de bore: | 9 bars à 20°C |
| — Température: | 80°C |
| — Durée: | 18 heures |

On recueille 20,2 g (rendement: 86%) d'un mélange de dichloro-2,4' et de dichloro-4,4' benzophénone brute.

Exemple 5

On procède comme à l'exemple 1 avec les composés et conditions suivants:

| | |
|---|---|
| — Acide fluorhydrique anhydre: | 100 g |
| — p-fluorotrichlorométhylbenzène: | 21,4 g 0,1 mole |
| — Fluorobenzène: | 9,6 g 0,1 mole |
| — Trifluorure de bore: | 10 bars à 20° C |
| — Température: | 100° C |
| — Durée: | 8 heures |

On recueille 19,2 g (rendement: 88%) d'un mélange de difluoro-4,4' et difluoro-2,4' benzophénone brute.

Exemple 6

On procède comme à l'exemple 1 avec les composés et conditions suivants:

| | |
|---|---|
| — Acide fluorhydrique anhydre: | 100 g |
| — Trifluorométhylbenzène: | 29,2 g 0,2 mole |
| — Bromobenzène: | 31,4 g 0,2 mole |
| — Trifluorure de bore: | 10 bars à 20° C |
| — Température: | 50° C |
| — Durée: | 4 heures |

On recueille 49 g (rendement: 93,8%) de bromo-4 benzophénone brute.

Exemple 7

On procède comme à l'exemple 1 avec les composés et conditions suivants:

| | |
|---|---|
| — Acide fluorhydrique: | 100 g |
| — Trifluorométhylbenzène: | 14,6 g 0,1 mole |
| — o-dichlorobenzène: | 22 g 0,15 mole |
| — Trifluorure de bore: | 10 bars à 20° C |
| — Température: | 60° C |
| — Durée: | 4 heures |

On recueille 24,9 g (rendement: 99,2%) d'un mélange de dichloro-2,3 et dichloro-3,4 benzophénone brute.

Exemple 8

On procède comme à l'exemple 1 avec les composés et conditions suivants:

| | |
|---|---|
| — Acide fluorhydrique anhydre: | 100 g |
| — Trifluorométhylbenzène: | 29,2 g 0,2 mole |
| — Trifluorure de bore: | 10 bars à 20° C |
| — Température: | 80° C |
| — Durée: | 6 heures |

On recueille 27 g (rendement: 54%) de trifluorométhyl-3 benzophénone brute.

Exemple 9

On procède comme à l'exemple 1 avec les composés et conditions suivants:

| | |
|---|---|
| — Acide fluorhydrique anhydre: | 100 g |
| — Trichloro-1,1,1 éthane: | 27 g 0,2 mole |
| — Orthodichlorobenzène: | 14,7 g 0,1 mole |
| — Trifluorure de bore: | 6 bars à 20° C |
| — Température: | 80° C |
| — Durée: | 24 heures |

On recueille 8,2 g (rendement: 43%) d'un mélange de dichloro-2,3 et dichloro-3,4 acétophénone brute.

## Revendications

1. Procédé de préparation de phénylcétones caractérisé en ce que, dans une première étape, on fait réagir un halogéno ou trihalogénométhylbenzène ayant pour formule générale:

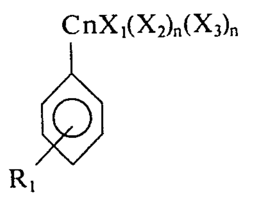

(I)

où:

- n est un nombre entier égal à 0 ou 1,
- $X_1$, $X_2$ et $X_3$ identiques ou différents représentent le chlore, le brome, l'iode ou le fluor et
- $R_1$ représente au moins un élément choisi parmi le groupe comprenant l'hydrogène, les radicaux alkyle et alkoxy ayant de 1 à 6 atomes de carbone, les radicaux phényle et phénoxy substitués par au moins un groupement plus désactivant que le groupement $CnX_1(X_2)_n(X_3)_n$, OH, Cl, Br, I et F,

avec un composé aliphatique ou aromatique trihalogénométhylé en présence de trifluorure de bore en quantité telle que la pression absolue de trifluorure de bore dans l'enceinte réactionnelle soit supérieure à 1 bar et en présence d'acide fluorhydrique comme solvant et, en ce que, dans une deuxième étape, on hydrolyse le produit résultant.

2. Procédé selon la revendication 1 caractérisé en ce que le composé aliphatique ou aromatique trihalogénométhylé a pour formule générale:

$$R_2-CX_4X_5X_6 \qquad (II)$$

où $R_2$ représente un radical aromatique ou aliphatique et $X_4X_5X_6$ identiques ou différents représentent Br, Cl, r, F.

3. Procédé selon la revendication 2 caractérisé en ce que $R_2$ représente un radical alkyle, phényle, alkylphényle, phénylalkyl ou phényle comportant au moins un substituant halogène, $NO_2$, CN, $NH_2$, COOH.

4. Procédé selon l'une quelconque des revendications précédentes caractérisé en ce que on utilise une quantité d'acide fluorhydrique telle que le rapport molaire de l'acide fluorhydrique à l'halogéno ou trihalogénométhylbenzène est compris entre 5 et 50.

5. Procédé selon l'une quelconque des revendications précédentes caractérisé en ce que l'acide fluorhydrique mis en oeuvre est de l'acide fluorhydrique anhydre.

6. Procédé selon l'une quelconque des revendications précédentes caractérisé en ce que l'halogéno ou trihalogénométhylbenzène et le composé de formule II sont utilisés en quantité sensiblement équimolaire.

7. Procédé selon l'une quelconque des revendications précédentes caractérisé en ce qu'on utilise une quantité de trifluorure de bore telle que la pression absolue de $BF_3$ dans l'enceinte réactionnelle soit comprise entre 6 et 20 bars.

8. Procédé selon l'une quelconque des revendications précédentes caractérisé en ce qu'on opère dans la première étape à une température comprise entre −20° C et 150° C.

9. Procédé selon l'une quelconque des revendications précédentes caractérisé en ce que l'on effectue la deuxième étape en milieu acide ou basique à une température comprise entre 0 et 80° C.

**Patentansprüche**

1. Verfahren zur Herstellung von Phenylketonen, dadurch gekennzeichnet, daß man in einer ersten Stufe ein Halogen- oder Trihalogenmethylbenzol mit der Formel

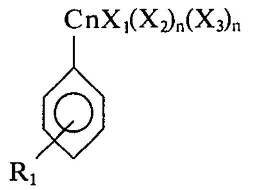

(I)

in der

n 0 oder 1 ist,
$X_1$, $X_2$ und $X_3$, gleich oder verschieden, Cl, Br, I oder F sind,
$R_1$ wenigstens ein Element, das aus der Gruppe ausgewählt ist, die Wasserstoff, die Alkyl- und Alkoxyradikale mit 1 bis 6 Kohlenstoffatomen, die mit wenigstens einer stärker desaktivierenden Gruppe als die Gruppe $CnX_1(X_2)_n(X_3)_n$, OH, Cl, Br, I und F substituierten Phenyl- und Phenoxyradikale umfaßt, bedeutet

mit einer aliphatischen oder aromatischen Trihalogenmethylverbindung in Gegenwart von Bortrifluorid in einer derartigen Menge, daß der absolute Druck des Bortrifluorids im Reaktionsraum über 1 bar liegt, und in Gegenwart von Fluorwasserstoffsäure als Lösungsmittel umsetzt und daß man in einer zweiten Stufe das erhaltene Produkt hydrolysiert.

2. Verfahren nach Anspruch 1, dadurch gekennzeichnet, daß die aliphatische oder aromatische Trihalogenmethylverbindung die allgemeine Formel hat

$$R_2{-}CX_4X_5X_6 \qquad (II)$$

in der $R_2$ ein aromatisches oder aliphatisches Radikal bedeutet und $X_4$, $X_5$ und $X_6$, gleich oder verschieden, Br, Cl oder F bedeuten.

3. Verfahren nach Anspruch 2, dadurch gekennzeichnet, daß $R_2$ ein Alkyl-, Phenyl-, Alkylphenyl-, Phenylalkyl- oder Phenylradikal mit wenigstens einem Halogen-, $NO_2$-, CN-, $NH_2$- oder COOH-Substituenten ist.

4. Verfahren nach einem der vorangehenden Ansprüche, dadurch gekennzeichnet, daß man eine derartige Menge Fluorwasserstoffsäure einsetzt, daß das Molverhältnis zwischen Fluorwasserstoffsäure und dem Halogen- oder Trihalogenmethylbenzol zwischen 5 und 50 liegt.

5. Verfahren nach einem der vorangehenden Ansprüche, dadurch gekennzeichnet, daß die eingesetzte Fluorwasserstoffsäure wasserfreie Fluorwasserstoffsäure ist.

6. Verfahren nach einem der vorangehenden Ansprüche, dadurch gekennzeichnet, daß das Halogen- oder Trihalogenmethylbenzol und die Verbindung der Formel II in ungefähr äquimolarer Menge eingesetzt werden.

7. Verfahren nach einem der vorangehenden Ansprüche, dadurch gekennzeichnet, daß man eine derartige Menge Bortrifluorid einsetzt, daß der absolute Druck des $BF_3$ im Reaktionsraum zwischen 6 und 20 bar liegt.

8. Verfahren nach einem der vorangehenden Ansprüche, dadurch gekennzeichnet, daß man in der ersten Stufe bei einer Temperatur zwischen −20° C und 150° C arbeitet.

9. Verfahren nach einem der vorangehenden Ansprüche, dadurch gekennzeichnet, daß man in der zweiten Stufe in saurem oder basischem Milieu bei einer Temperatur zwischen 0 und 80° C arbeitet.

## Claims

1. Process for preparing phenyl ketones, characterised in that, in a first stage, a halo- or trihalomethylbenzene having the general formula:

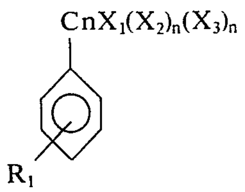

(I)

where:

- n is an integer equal to 0 or 1,
- $X_1$, $X_2$ and $X_3$, which are identical or different, denote chlorine, bromine, jodine or fluorine and
- $R_1$ denotes at least one member chosen from the group comprising hydrogen, the alkyl and alkoxy radicals containing from 1 to 6 carbon atoms, the phenyl and phenoxy radicals substituted by at least one group which is more deactivating than the group $CnX_1(X_2)_n(X_3)_n$, OH, Cl, Br, I and F,

is reacted with an aliphatic or aromatic trihalomethyl compound in the presence of boron trifluoride in such quantity that the absolute pressure of boron trifluoride in the reaction enclosure is greater than 1 bar and in the presence of hydrofluoric acid as a solvent and, in that, in a second stage, the resulting product is hydrolysed.

2. Process according to Claim 1, characterised it that the aliphatic or aromatic trihalomethyl compound has the general formula:

$$R_2-CX_4X_5X_6 \qquad (II)$$

where $R_2$ denotes an aromatic or aliphatic radical, and $X_4$, $X_5$ and $X_6$, which are identical or different, denote Br, Cl, or F.

3. Process according to Claim 2, characterised in that $R_2$ denotes an alkyl, phenyl, alkylphenyl or phenylalkyl radical, or a phenyl radical containing at least one substituent halogen, $NO_2$, CN, $NH_2$, or COOH.

4. Process according to any one of the preceding claims, characterised in that a quantity of hydrofluoric acid is employed such that the molar ratio of hyrofluoric acid to the halo or trihalomethylbenzene is between 5 and 50.

5. Process according to any one of the preceding claims, characterised in that the hydrofluoric acid employed is anhydrous hydrofluoric acid.

6. Process according to any one of the preceding claims, characterised in that the halo- or trihalomethylbenzene and the compound of formula II are employed in substantially equimolar quantities.

7. Process according to any one of the preceding claims, characterised in that a quantity of boron trifluoride is employed such that the absolute pressure of $BF_3$ in the reaction enclosure is between 6 and 20 bars.

8. Process according to any one of the preceding claims, characterised in that, in the first stage, it is operated at a temperature of between −20° C and 150° C.

9. Process according to any one of the preceding claims, characterised in that the second stage is carried out in an acidic or basic medium at a temperature of between 0 and 80° C.